# EUROPEAN PATENT APPLICATION

(11) **EP 4 703 720 A1**
(43) Date of publication of application: **04.03.2026**
(21) Application number: 24796677.3
(22) Date of filing: 27.03.2024
(51) Int. Cl.: G01N 27/62, C12Q 1/68

(54) **OLIGONUCLEOTIDE ANALYSIS METHOD**

(30) Priority: 28.04.2023 JP 2023074120
(71) Applicant: SHIMADZU CORPORATION, Kyoto-shi, Kyoto 604-8511 (JP)
(72) Inventor: NISHIKAZE, Takashi, Kyoto-shi, Kyoto 604-8511 (JP)
(74) Representative: Müller-Boré & Partner Patentanwälte PartG mbB
(86) International application number: PCT/JP2024/012140
(87) International publication number: WO 2024/224919

(57) **Abstract**

A method for analyzing an oligonucleotide, the method including: an application step of applying a sample containing the oligonucleotide and a matrix to a plate; an acquisition step of performing mass spectrometry by irradiating the sample applied to the plate with a laser beam in a MALDI mass spectrometer to acquire a mass spectrum; and a determination step of, when a peak other than a peak derived from the oligonucleotide is observed in the acquired mass spectrum, determining that the sample may contain an impurity, wherein the intensity of the laser beam is an intensity that can suppress in-source decay of the oligonucleotide, and the matrix includes an acetophenone-based compound.

## Description

### TECHNICAL FIELD

The present invention relates to a method for analyzing an oligonucleotide.

### BACKGROUND ART

In recent years, the application of synthetic oligonucleotides to the medical field has been advancing. Examples include ribozymes, nucleic acid aptamers, antisense oligonucleotides, and RNA interference (RNAi), which are called nucleic acid drugs. Synthetic oligonucleotides are mainly synthesized by a continuous reaction using a phosphoramidite nucleic acid monomer on a solid-phase support.

### CITATION LIST

### PATENT LITERATURE

[Patent Literature 1] JP 2016-057219 A

### SUMMARY OF INVENTION

### TECHNICAL PROBLEM

Although methods for synthesizing oligonucleotides have been optimized to a level where purification is unnecessary for use in applications such as PCR primers, more advanced purification and analysis are required for application to pharmaceuticals. In particular, long-chain DNA, modified forms, and backbone-modified forms are prone to unreacted coupling or side reactions during synthesis. Therefore, the reaction mixture of a synthetic oligonucleotide may mainly contain incompletely elongated oligonucleotides or incomplete oligonucleotides. Furthermore, incompletely or incorrectly deprotected fragments and stereoisomers with partial chirality may also be included as impurities.

For the analysis of such a reaction mixture of a synthetic oligonucleotide, high-performance liquid chromatography (HPLC) or liquid chromatography-mass spectrometry (LC-MS) is mainly used (for example, Patent Literature 1).

However, when analyzing by HPLC or the like, it takes time to separate each component, leaving room for improvement from the viewpoint of throughput. In addition, since there are many side components such as stereoisomers whose properties are similar to the main component, it is necessary to change the separation mode according to the type of impurity. Therefore, another problem is that the workload for analysis is high compared to general small-molecule drugs.

The present invention has been made in view of the above circumstances, and an object thereof is to provide a method for analyzing an oligonucleotide that can easily detect impurities.

### SOLUTION TO PROBLEM

As a result of diligent research, the present inventors have found that by performing MALDI mass spectrometry using a predetermined matrix and setting a predetermined laser intensity, impurities contained in the oligonucleotide to be analyzed can be easily detected, and have completed the present invention.

A first aspect of the present invention relates to a method for analyzing an oligonucleotide, the method including:
an application step of applying a sample containing the oligonucleotide and a matrix to a plate;
an acquisition step of performing mass spectrometry by irradiating the sample applied to the plate with a laser beam in a MALDI mass spectrometer to acquire a mass spectrum; and
a determination step of, when a peak other than a peak derived from the oligonucleotide is observed in the acquired mass spectrum, determining that the sample may contain an impurity, wherein
the intensity of the laser beam is an intensity that can suppress in-source decay of the oligonucleotide, and
the matrix includes an acetophenone-based compound.

### ADVANTAGEOUS EFFECTS OF INVENTION

According to the present invention, it is possible to provide a method for analyzing an oligonucleotide that can easily detect impurities.

### BRIEF DESCRIPTION OF DRAWINGS

[FIG. 1] FIG. 1 is a mass spectrum obtained by analyzing an oligonucleotide with a MALDI mass spectrometer. The horizontal axis indicates the m/z value, and the vertical axis indicates the detection intensity.
[FIG. 2] FIG. 2 is a mass spectrum obtained by analyzing an oligonucleotide with a MALDI mass spectrometer. The horizontal axis indicates the m/z value, and the vertical axis indicates the detection intensity.
[FIG. 3] FIG. 3 is a mass spectrum obtained by analyzing an oligonucleotide with a MALDI mass spectrometer. The horizontal axis indicates the m/z value, and the vertical axis indicates the detection intensity.
[FIG. 4] FIG. 4 is a mass spectrum obtained by analyzing an oligonucleotide with a MALDI mass spectrometer. The horizontal axis indicates the m/z value, and the vertical axis indicates the detection intensity.
[FIG. 5] FIG. 5 is a mass spectrum obtained by analyzing an oligonucleotide with a MALDI mass spectrometer. The horizontal axis indicates the m/z value, and the vertical axis indicates the detection intensity.

### DESCRIPTION OF EMBODIMENTS

Hereinafter, an embodiment of the present invention (hereinafter referred to as "the present embodiment") will be described. However, the present embodiment is not limited to this. In this specification, the notation "A to Z" means a range including the upper and lower limits (i.e., A or more and Z or less), and when no unit is described for A and a unit is described only for Z, the unit of A is the same as the unit of Z.

### « Method for Analyzing Oligonucleotide»

A first aspect of the present invention is a method for analyzing an oligonucleotide, the method including:
an application step of applying a sample containing the oligonucleotide and a matrix to a plate;
an acquisition step of performing mass spectrometry by irradiating the sample applied to the plate with a laser beam in a MALDI mass spectrometer to acquire a mass spectrum; and
a determination step of, when a peak other than a peak derived from the oligonucleotide is observed in the acquired mass spectrum, determining that the sample may contain an impurity, wherein
the intensity of the laser beam is an intensity that can suppress in-source decay of the oligonucleotide, and
the matrix includes an acetophenone-based compound.

### <Application Step>

In this step, a sample containing an oligonucleotide and a matrix are applied to a plate.

In the present embodiment, the sample to be analyzed contains an oligonucleotide. "Oligonucleotide" means a polymer of 2 to 50 identical or different nucleotides linked by a phosphodiester bond, a phosphorodithioate bond, or the like. The oligonucleotide is usually composed of deoxyribonucleotides and ribonucleotides as constituent units. Examples of the nucleic acid base contained in the deoxyribonucleotide and ribonucleotide include adenine (A), guanine (G), cytosine (C), thymine (T), and uracil (U). The nucleic acid base may be a nucleic acid base modified with a methyl group, a methoxyethyl group, or the like. It may also be a bridged nucleic acid called a locked nucleic acid, or a morpholino nucleic acid. The oligonucleotide may usually be in a single-stranded form or a double-stranded form. In addition, the oligonucleotide may be one in which another compound, for example, a sugar such as GalNAc, is bonded to its terminus.

In the present embodiment, the sample is not particularly limited as long as it contains the target oligonucleotide, and it may be in a solution state or a solid state. In one aspect of the present embodiment, the sample is preferably an aqueous solution.

"Matrix" means a compound that assists in the ionization of the oligonucleotide in MALDI mass spectrometry. In the present embodiment, the matrix includes an acetophenone-based compound. Here, "acetophenone-based compound" means a compound having the chemical structure of acetophenone, and examples thereof include acetophenone, hydroxyacetophenone (HAP), dihydroxyacetophenone (DHAP), and trihydroxyacetophenone (THAP). In the present embodiment, the acetophenone-based compound preferably includes at least one selected from the group consisting of hydroxyacetophenone, dihydroxyacetophenone, and trihydroxyacetophenone. The matrix may contain a compound other than the acetophenone-based compound as long as the effects of the present invention are exerted.

Conventionally, when performing MALDI mass spectrometry of an oligonucleotide, the analysis has been performed using a matrix that easily causes in-source decay (for example, 3-HPA). This is because the sequence information of the oligonucleotide can be determined by mass-analyzing the in-source decayed fragment group (sometimes called an "ion series"). Therefore, there has been no idea of intentionally suppressing in-source decay to perform MALDI mass spectrometry of an oligonucleotide. The present inventor, without being bound by conventional technical common sense, tried to perform MALDI mass spectrometry of an oligonucleotide using a matrix containing an acetophenone-based compound and a laser beam of a predetermined intensity described later, and found that the appearance of "peaks derived from the in-source decayed fragment group" was suppressed, and that impurities contained in the oligonucleotide to be analyzed could be easily detected, thereby completing the present invention.

In one aspect of the present embodiment, the matrix preferably further contains ammonium citrate. By doing so, suppression of salt addition such as sodium addition becomes possible.

The method of applying the sample and the matrix to the plate is not particularly limited, and a known method can be adopted. For example, a method may be mentioned in which an aqueous solution of the sample and a solution of the matrix are mixed, the mixture is dropped onto the plate, and then the solvent is evaporated by natural drying or the like.

In one aspect of the present embodiment, the application step preferably includes applying a solution containing the sample and the matrix to the plate on which microcrystals of the matrix have been applied. By doing so, the sensitivity and quantitativity of the mass spectrometry are improved. As a method for applying the microcrystals of the matrix to the plate, for example, the following method may be mentioned. First, a solution in which the matrix is dissolved in an organic solvent (for example, methanol, ethanol, etc.) is dropped onto the plate. Thereafter, the organic solvent is evaporated by natural drying to form microcrystals of the matrix on the plate. Here, instead of dropping the matrix solution onto the plate, the solution may be sprayed onto the plate by a spray. The spray is not particularly limited as long as it can spray uniformly, and may be an airbrush or a mist spray. Instead of the spray, a dedicated device for spraying a matrix (for example, iMLayer AERO, manufactured by Shimadzu Corporation) or a matrix deposition device (for example, iMLayer, manufactured by Shimadzu Corporation) may be used.

### <Acquisition Step>

In this step, in a MALDI mass spectrometer, the sample applied to the plate is irradiated with a laser beam to perform mass spectrometry and acquire a mass spectrum.

In the present embodiment, the MALDI mass spectrometer is not particularly limited as long as it is an apparatus capable of performing mass spectrometry by matrix-assisted laser desorption/ionization. As the MALDI mass spectrometer, for example, a benchtop MALDI-TOF MS "MALDI-8030" (manufactured by Shimadzu Corporation) may be mentioned.

In the present embodiment, the laser beam with which the sample is irradiated is preferably an ultraviolet laser beam. The intensity of the laser beam is an intensity that can suppress in-source decay of the oligonucleotide. Here, the term "intensity that can suppress in-source decay" does not only mean "an intensity that can completely prevent in-source decay" but is a concept that includes the meaning of "an intensity at which some in-source decay may occur." In one aspect of the present embodiment, the intensity of the laser beam is preferably an intensity that can suppress in-source decay of the oligonucleotide when a matrix containing an acetophenone-based compound is used. "In-source decay" means all fragmentation that occurs in the ionization source simultaneously with or immediately after ionization.

In one aspect of the present embodiment, the intensity of the laser beam is preferably an intensity slightly higher than the threshold at which the oligonucleotide is ionized. The "threshold at which the oligonucleotide is ionized" can also be understood as the intensity value when the ion of the oligonucleotide is detected by gradually increasing the intensity of the laser beam. When the intensity of the laser beam is equal to or higher than the threshold, the ion can be detected no matter where on the sample spot on the plate the laser beam is applied. In another aspect of the present embodiment, the intensity of the laser beam can also be understood as being an intensity slightly higher than the threshold at which the oligonucleotide is ionized when a matrix containing an acetophenone-based compound is used.

For example, when using a benchtop MALDI-TOF MS "MALDI-8030" (manufactured by Shimadzu Corporation) as the MALDI mass spectrometer, the "intensity slightly higher than the threshold at which the oligonucleotide is ionized" can be determined by the following procedure. First, the lowest laser power at which ions start to appear is identified, then peaks that are thought to be from in-source decay, which are generated by measuring at an extremely increased laser power from there, are searched for, and finally, the laser power is lowered to a level that suppresses the in-source decay peaks while still allowing a stable mass spectrum to be obtained, thereby determining the threshold. Alternatively, the threshold is determined by gradually increasing the laser power from a low laser power at which no ions are detected and performing measurement, and searching for the lowest laser power at which the S/N ratio of the oligonucleotide peak becomes stable. For other MALDI mass spectrometers, the set value of the laser beam intensity can be determined by similar methods.

The position to irradiate with the laser beam is not particularly limited as long as it is within the sample spot on the plate, and may be anywhere.

The range of the mass spectrum to be acquired (m/z range) is not particularly limited as long as the peak of the oligonucleotide and the peak of impurities can be detected, but for example, it is 1 to 20000. For the purpose of excluding peaks derived from the matrix, a range of 500 to 20000 or 1000 to 20000 may be used.

### <Determination Step>

In this step, when a peak other than the peak derived from the oligonucleotide is observed in the acquired mass spectrum, it is determined that the sample may contain an impurity. In one aspect of the present embodiment, when a peak other than the peak derived from the oligonucleotide is observed, it may be determined that the sample contains an impurity.

In the present embodiment, "peak derived from the oligonucleotide" means a peak derived from the oligonucleotide that is the main object of measurement (oligonucleotide that is the main product, or oligonucleotide of the main component). Examples of the peak derived from the oligonucleotide include a peak of the full-length oligonucleotide and a peak of the oligonucleotide that has undergone in-source decay. In one aspect of the present embodiment, the peak derived from the oligonucleotide preferably includes a peak of the full-length oligonucleotide and a peak of the oligonucleotide that has undergone in-source decay. The peak of the full-length oligonucleotide can be identified from the molecular weight of the oligonucleotide. The peak of the oligonucleotide that has undergone in-source decay can be identified by performing MALDI mass spectrometry on a standard product of the oligonucleotide (purity of 90% or more is preferable) under conditions where in-source decay occurs (for example, the conditions of the comparative example described later).

In the present embodiment, the "peak other than the peak derived from the oligonucleotide" is a peak derived from an impurity. Therefore, when this peak is observed, it can be determined that the sample may contain an impurity. The impurity may be a deletion product or an addition product of the oligonucleotide.

Examples of the deletion product of the oligonucleotide include deletion products in which 1 to 14 bases are deleted, and preferably deletion products in which 1 to 9 bases are deleted. The location where the deletion of the deletion product occurs is not particularly limited, but it often occurs at the 5' end or the 3' end. In some cases, only the base part of the nucleotide is deleted.

Examples of the addition product of the oligonucleotide include an addition product in which a protecting group (for example, a dimethoxytrityl group (DMTr group)) remains at the 5' end, an addition product in which an acetyl group used for capping remains at the 5' end, an addition product in which 1 to 3 bases are added, or an impurity generated by the capping reaction acting on an internal nucleotide residue.

### <Other Steps>

### <Impurity Quantitation Step>

In one aspect of the present embodiment, the method for analyzing an oligonucleotide preferably further includes an impurity quantitation step of quantitating the impurity based on the intensity of a peak other than the peak derived from the oligonucleotide. The method for quantitating the impurity is not particularly limited, and a known method can be used. Examples of such methods include an absolute calibration curve method, an internal standard method, and relative quantitation with respect to the main product.

The method for analyzing an oligonucleotide according to the present embodiment has been described above. The analysis method performs MALDI mass spectrometry under conditions where in-source decay is suppressed, compared to conventional analysis methods. Therefore, impurities contained in the oligonucleotide to be analyzed can be easily detected. The method for analyzing an oligonucleotide according to the present embodiment can also be used for quality control of nucleic acid drugs.

### EXAMPLES

Hereinafter, the present invention will be described in more detail with reference to examples, but the present invention is not limited thereto.

### «Experiment 1: Examination of Matrix Components»

In the analysis of oligonucleotides by MALDI mass spectrometry, the following experiment was conducted to investigate the influence of matrix components. First, an aqueous solution of Mipomersen (concentration 0.1 mg/mL) was prepared as a sample.

Next, the following two types of aqueous solutions were prepared as matrix solutions.
(1) 3-HPA (3-hydroxypicolinic acid, final concentration: 40 mg/mL), ACD (Ammonium Citrate Dibasic, final concentration: 40 mM) in 50% aqueous acetonitrile
(2) THAP (trihydroxyacetophenone) (final concentration: 40 mg/mL), ACD (final concentration: 40 mM) in 50% aqueous MeOH

The sample and the matrix solution ((1) or (2)) were mixed at a volume ratio of 1:1 to obtain a mixed solution. 1.5 µL of the mixed solution was dropped onto a MALDI plate and air-dried. Thereafter, the MALDI plate was set in a benchtop MALDI-TOF MS apparatus (product name: MALDI-8030, manufactured by Shimadzu Corporation), and MALDI mass spectrometry was performed under the following measurement conditions. The intensity of the laser beam described below is an intensity that can suppress in-source decay of the oligonucleotide when a matrix containing THAP (an acetophenone-based compound) is used.

### Measurement Conditions

Ion mode: Negative ion linear mode
Laser beam intensity: Variable depending on the matrix, slightly exceeding the threshold
Delayed extraction: Optimized for 7000 Da

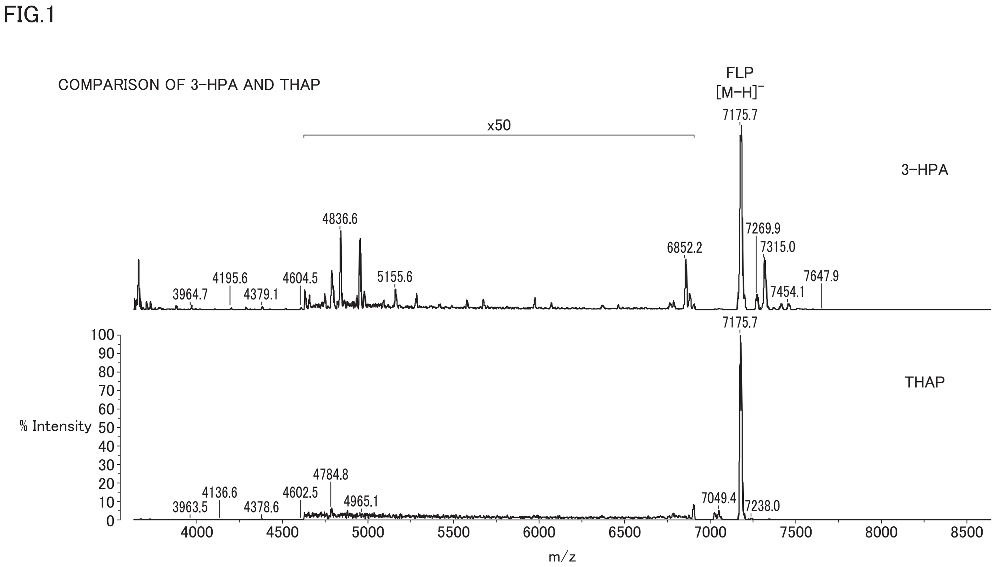

FIG. 1 shows the mass spectrum obtained by MALDI mass spectrometry. In FIG. 1, the range indicated by "×50" shows peaks with the peak intensity magnified 50 times. From the results in FIG. 1, when 3-HPA was used, in-source decay (ISD) peaks were observed in the range where the peak intensity was magnified 50 times. On the other hand, when THAP was used, almost no peaks were observed in this range. From the above results, it was found that when an acetophenone-based compound such as THAP is used as a matrix, mass spectrometry can be performed while suppressing in-source decay.

### «Experiment 2: Mass Spectrometry of Oligonucleotide Containing Impurities »

Based on the results of Experiment 1, the following experiment was conducted to examine whether impurities contained in a sample can be detected when THAP is used as a matrix. First, the following three types of aqueous solutions were prepared as samples. Here, "impurities" mean a 1-base deletion product (1 base on the 3' end side is deleted) and a 3-base deletion product (3 bases on the 3' end side are deleted) of each oligonucleotide.
(1) Mipomersen (concentration 0.1 mg/mL), impurities (10% by mass each, based on Mipomersen contained in the sample)
(2) Nusinersen (concentration 0.1 mg/mL), impurities (10% by mass each, based on Nusinersen contained in the sample)
(3) Inotersen (concentration 0.1 mg/mL), impurities (10% by mass each, based on Inotersen contained in the sample)

Table 1 shows information on the oligonucleotides contained in the samples.

**[Table 1]**

| Name | Chemical formula | Mw | Note |
|---|---|---|---|
| Mipomersen | C230H324N67O122P19S19 | 7177.2 | ASO, PS (full), 2'-MOE, 20 mer |
| 5'-G*-mC*-mC*-mU*-mC*-dA-dG-dT-dmC-dT-dG-dmC-dT-dT-dmC-G*-mC*-A*-mC*-mC*-3' | | | |
| Nusinersen | C234H340N61O128P17S17 | 7127.2 | ASO, PS (full), 2'-MOE, 18 mer |
| 5'-T*-mC*-A*-mC*-T*-T*-T*-mC*-A*-T*-A*-A*-T*-G*-mC*-T*-G*-G*-3' | | | |
| Inotersen | C230H318N69O121P19S19 | 7183.1 | ASO, PS (full), 2'-MOE, 20 mer |
| 5'-T*-mC*-T*-T*-G*-dG-dT-dT-dA-dmC-dA-dT-dG-dA-dA-A*-T*-mC*-mC*-mC*-3' | | | |

| | | | |
|---|---|---|---|
| * = 2'-O-(2-methoxyethyl); m = 5-methyl; d = 2'-deoxy; PS = phosphorothioate | | | |

The following aqueous solution was prepared as a matrix solution.
THAP (final concentration: 10 mg/mL), ACD (final concentration: 40 mM) in 70% aqueous MeOH

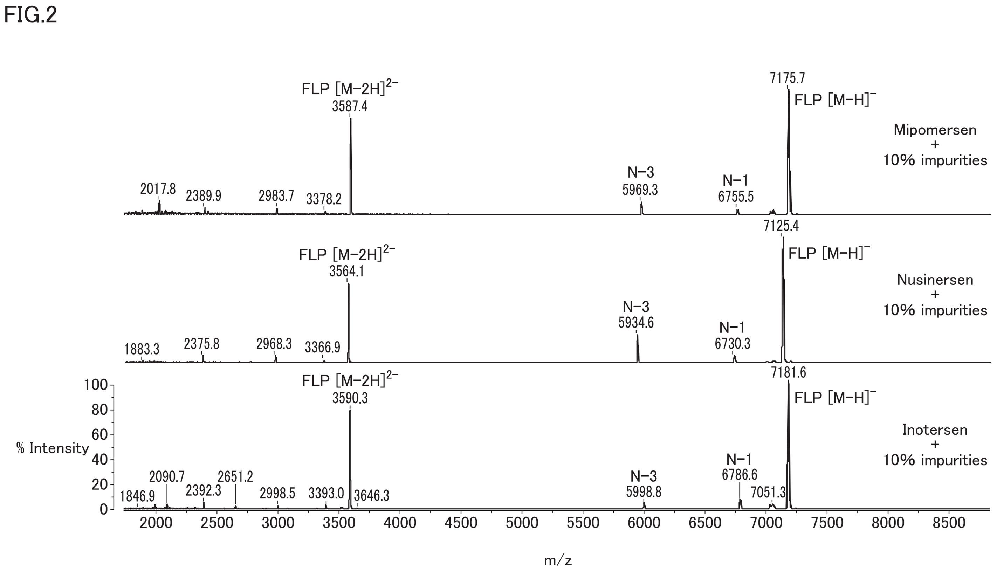

0.2 µL of a methanol solution of THAP (concentration: 10 mg/mL) was dropped onto a MALDI plate to form a film-like microcrystal. 1.5 µL of a mixed solution of the sample and the matrix solution (volume ratio of 1:1) was dropped onto the MALDI plate on which the microcrystal was formed, and air-dried (application step). Thereafter, the MALDI plate was set in a benchtop MALDI-TOF MS apparatus (product name: MALDI-8030, manufactured by Shimadzu Corporation), and MALDI mass spectrometry was performed under the same measurement conditions as in Experiment 1 to acquire a mass spectrum (acquisition step). The results are shown in FIG. 2.

From the results in FIG. 2, for all of Mipomersen, Nusinersen, and Inotersen, peaks other than the peaks derived from each oligonucleotide were observed, and it was found that these peaks were the peak of the 1-base deletion product (N-1) and the peak of the 3-base deletion product (N-3) (peaks of impurities) corresponding to each oligonucleotide (determination step).

### «Experiment 3: Detectable Concentration of Impurities»

The following experiment was conducted to examine to what concentration impurities contained in a sample can be detected. First, the following aqueous solution was prepared as a sample. Here, "impurities" mean a 1-base deletion product and a 3-base deletion product of the oligonucleotide.
Mipomersen (concentration 0.1 mg/mL), impurities (0 to 10% by mass based on Mipomersen contained in the sample)

The following aqueous solution was prepared as a matrix solution.
THAP (final concentration: 10 mg/mL), ACD (final concentration: 40 mM) in 70% aqueous MeOH

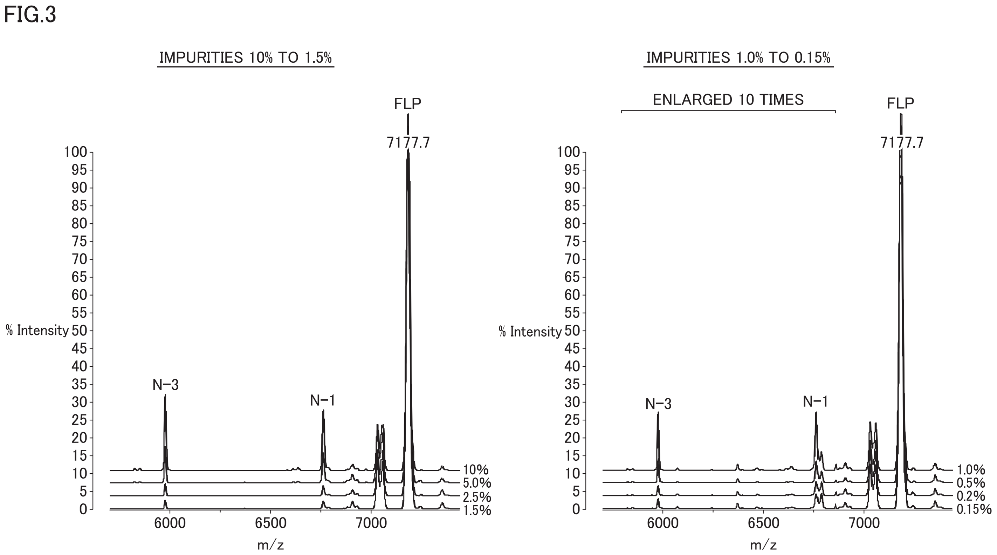

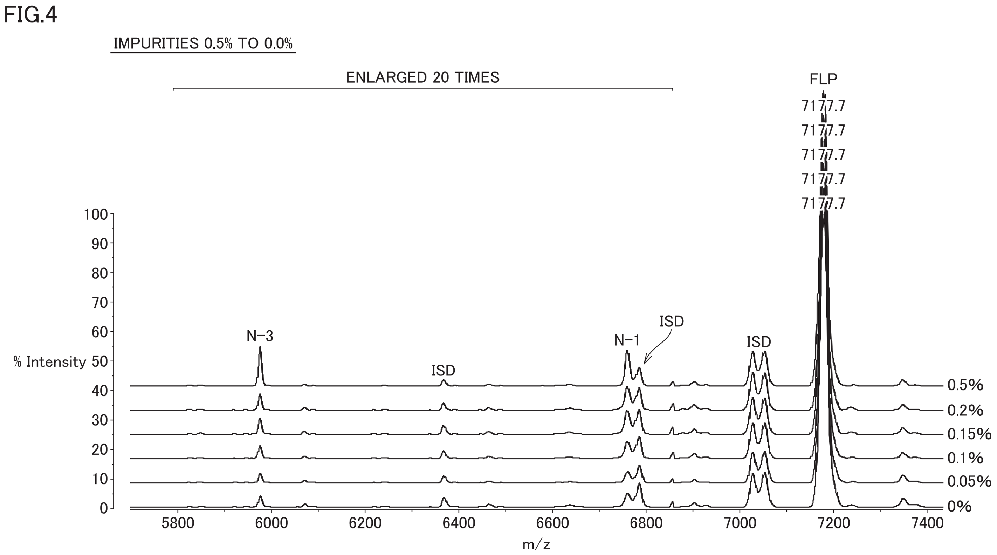

Using each of the prepared samples and the matrix solution, MALDI mass spectrometry was performed in the same manner as in Experiment 2 to acquire a mass spectrum. The results are shown in FIG. 3 and FIG. 4. **In** FIG. 3 and FIG. 4, the range described as "Enlarged 10 times" or "Enlarged 20 times" shows peaks with the peak intensity magnified 10 times or 20 times. From the results of FIG. 3 and FIG. 4, it was found that the impurities could be detected in a proportion range of 0.5% by mass to 10% by mass. Although some peaks overlapped with the peaks of in-source decayed fragments, the intensity was greater than the peak at the corresponding position in the mass spectrum when the impurity was 0% by mass. This suggested that the peak also included a peak derived from the impurity. In other words, it was suggested that by comparing the mass spectrum acquired from a standard oligonucleotide product (an oligonucleotide not containing impurities) and the mass spectrum acquired from a measurement sample, it can be determined whether an impurity with the same molecular weight as an in-source decayed fragment is contained.

### « Experiment 4: Analysis of Crude Product of Synthetic Oligonucleotide»

In Experiments 2 and 3, mass spectrometry was performed on samples containing impurities with known content ratios, but it was verified what kind of impurities would be detected when similar mass spectrometry was performed on a crude product of a synthetic oligonucleotide. First, Nusinersen was synthesized by a general phosphoramidite method using a solid-phase synthesizer to obtain a crude product. An aqueous solution of the obtained crude product (concentration: 0.5 mg/mL) was prepared and used as a sample.

The following aqueous solution was prepared as a matrix solution.
THAP (final concentration: 10 mg/mL), ACD (final concentration: 40 mM) in 70% aqueous MeOH

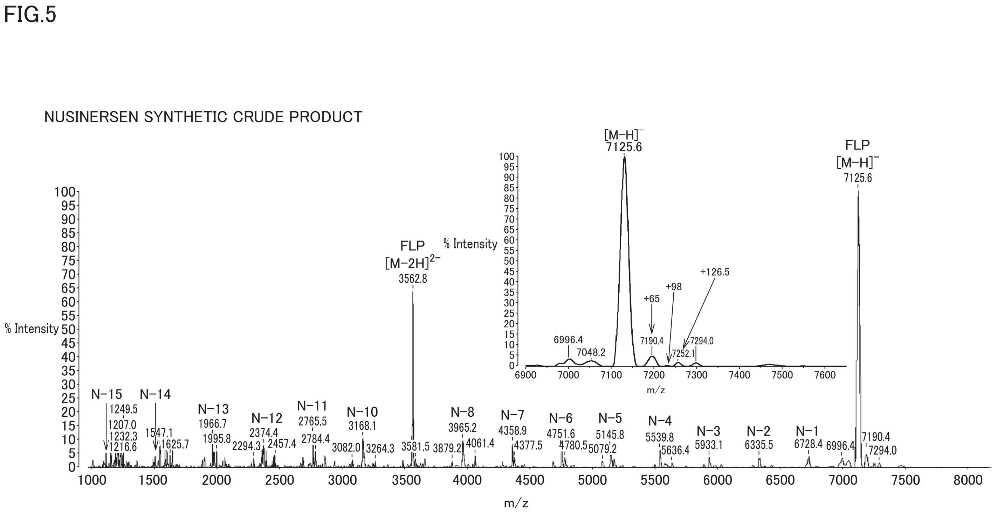

400 µL of a methanol solution of THAP (concentration: 10 mg/mL) was sprayed onto a MALDI plate with a sprayer to form a microcrystal. 1.5 µL of a mixed solution of the sample and the matrix solution (volume ratio of 1:1) was dropped onto the MALDI plate on which the microcrystal was formed, and air-dried (application step). Thereafter, the MALDI plate was set in a benchtop MALDI-TOF MS apparatus (product name: MALDI-8030, manufactured by Shimadzu Corporation), and MALDI mass spectrometry was performed under the same measurement conditions as in Experiment 1 to acquire a mass spectrum (acquisition step). The results are shown in FIG. 5.

In FIG. 5, in addition to the peaks of full-length Nusinersen ([M-H]⁻, [M-2H]²⁻), N-X series (N-1 to N-15), which are deletion products on the 5' end side, were detected in the low molecular weight region. In addition, deletion product groups on the 3' end side were also detected, although weakly. In the high molecular weight region, peaks thought to be impurities derived from capping and impurities with remaining protecting groups were also observed. From the above results, it was found that the method for analyzing an oligonucleotide according to the present invention can easily detect impurities.
It should be noted that although the crude synthetic product this time did not contain nucleotide addition products, it is known that addition products may also be generated depending on the synthesis conditions. Considering that the N-1 deletion product is detected at 0.5% by mass or more, it is thought that the N+1 addition product can also be detected in a similar concentration range.

### [Aspects]

It is understood by those skilled in the art that the plurality of exemplary embodiments and examples described above are specific examples of the following aspects.

(Item 1) A method for analyzing an oligonucleotide according to one aspect includes: an application step of applying a sample containing the oligonucleotide and a matrix to a plate; an acquisition step of performing mass spectrometry by irradiating the sample applied to the plate with a laser beam in a MALDI mass spectrometer to acquire a mass spectrum; and a determination step of, when a peak other than a peak derived from the oligonucleotide is observed in the acquired mass spectrum, determining that the sample may contain an impurity, wherein the intensity of the laser beam is an intensity that can suppress in-source decay of the oligonucleotide, and the matrix includes an acetophenone-based compound. According to the method for analyzing an oligonucleotide of Item 1, impurities can be easily detected.

(Item 2) In the method for analyzing an oligonucleotide according to Item 1, the acetophenone-based compound includes at least one selected from the group consisting of hydroxyacetophenone, dihydroxyacetophenone, and trihydroxyacetophenone. According to the method for analyzing an oligonucleotide of Item 2, in-source decay is further suppressed, and the detection sensitivity of impurities is improved.

(Item 3) In the method for analyzing an oligonucleotide according to Item 1 or 2, the impurity is a deletion product or an addition product of the oligonucleotide. According to the method for analyzing an oligonucleotide of Item 3, the analysis method is suitably used for detecting a deletion product or an addition product of the oligonucleotide.

(Item 4) In the method for analyzing an oligonucleotide according to any one of Items 1 to 3, the peak derived from the oligonucleotide includes a peak of the full-length oligonucleotide and a peak of the oligonucleotide that has undergone in-source decay.

(Item 5) In the method for analyzing an oligonucleotide according to any one of Items 1 to 4, the application step includes applying a solution containing the sample and the matrix to the plate on which microcrystals of the matrix have been applied. According to the method for analyzing an oligonucleotide of Item 5, the detection sensitivity and quantitativity of impurities are improved.

(Item 6) In the method for analyzing an oligonucleotide according to any one of Items 1 to 5, the matrix further includes ammonium citrate. According to the method for analyzing an oligonucleotide of Item 6, the addition of sodium ions to the oligonucleotide is suppressed, and the detection accuracy is improved.

(Item 7) The method for analyzing an oligonucleotide according to any one of Items 1 to 6, further quantitating the impurity based on the intensity of a peak other than the peak derived from the oligonucleotide. According to the method for analyzing an oligonucleotide of Item 7, quantitation of impurities becomes possible.

Although the embodiments and examples of the present invention have been described above, it is originally planned to appropriately combine the configurations of the above-described embodiments and examples.

The embodiments and examples disclosed this time should be considered illustrative in all respects and not restrictive. The scope of the present invention is indicated by the claims rather than the above-described embodiments and examples, and it is intended that all changes within the meaning and range equivalent to the claims be included.

## Claims

1. A method for analyzing an oligonucleotide, the method comprising:
an application step of applying a sample containing the oligonucleotide and a matrix to a plate;
an acquisition step of performing mass spectrometry by irradiating the sample applied to the plate with a laser beam in a MALDI mass spectrometer to acquire a mass spectrum; and
a determination step of, when a peak other than a peak derived from the oligonucleotide is observed in the acquired mass spectrum, determining that the sample may contain an impurity, wherein
the intensity of the laser beam is an intensity that can suppress in-source decay of the oligonucleotide, and
the matrix includes an acetophenone-based compound.

2. The method for analyzing an oligonucleotide according to claim 1, wherein the acetophenone-based compound includes at least one selected from the group consisting of hydroxyacetophenone, dihydroxyacetophenone, and trihydroxyacetophenone.

3. The method for analyzing an oligonucleotide according to claim 1 or 2, wherein the impurity is a deletion product or an addition product of the oligonucleotide.

4. The method for analyzing an oligonucleotide according to claim 1 or 2, wherein the peak derived from the oligonucleotide includes a peak of the full-length oligonucleotide and a peak of the oligonucleotide that has undergone in-source decay.

5. The method for analyzing an oligonucleotide according to claim 1 or 2, wherein the application step includes applying a solution containing the sample and the matrix to the plate on which microcrystals of the matrix have been applied.

6. The method for analyzing an oligonucleotide according to claim 1 or 2, wherein the matrix further includes ammonium citrate.

7. The method for analyzing an oligonucleotide according to claim 1 or 2, further comprising an impurity quantitation step of quantitating the impurity based on the intensity of a peak other than the peak derived from the oligonucleotide.
